(19) Europäisches Patentamt
European Patent Office
Office européen des brevets

(11) Publication number: 0 027 993 A1

(12) **EUROPEAN PATENT APPLICATION**

(21) Application number: 80106408.0

(22) Date of filing: 21.10.80

(51) Int. Cl.³: C 07 C 101/77, C 07 C 101/72, A 61 K 31/195, A 61 K 31/215

(30) Priority: 22.10.79 US 86871
27.05.80 US 153388

(43) Date of publication of application: 06.05.81 Bulletin 81/18

(84) Designated Contracting States: AT BE CH DE FR GB IT LI LU NL SE

(71) Applicant: MERCK & CO. INC., 126, East Lincoln Avenue P.O. Box 2000, Rahway, New Jersey 07065 (US)

(72) Inventor: Kollonitsch, Janos, 3 Plymouth Road, Westfield, New Jersey 07090 (US)

(74) Representative: Abitz, Walter, Dr.-Ing. et al, Abitz, Morf, Gritschneder P.O. Box 86 01 09, D-8000 München 86 (DE)

(54) **6-Fluorodihydroxyphenylalanines, a process for preparing and a pharmaceutical composition containing the same.**

(57) Novel 6-Fluorohydroxyphenylalanines, their preparation and pharmaceutical compositions containing the same are disclosed.

The novel 6-Fluorohydroxyphenylalanines have the formula

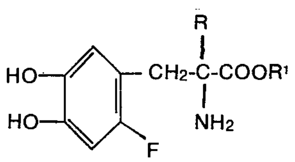

and pharmaceutically acceptable salts thereof wherein

R is H or $CH_3$ and

$R^1$ is H, $C_1$-$C_{18}$ alkyl or substituted $C_1$-$C_3$ alkyl.

EP 0 027 993 A1

Title

6-FLUORODIHYDROXYPHENYLALANINES, A PROCESS FOR PREPARING AND A PHARMACEUTICAL COMPOSITION CONTAINING THE SAME

## Background of the Invention

The present invention is concerned with 6-fluorodihydroxyphenylalanines.

4-Halohydroxyphenylalanines are described in U.S. 3,344,023. 2-Methyl-3-(2-fluoro-3,4-dihydroxyphenyl) alanine is disclosed in J. Med. Chem. 9, 738 (1966).

6-Fluoro-3,4-dihydroxyphenylalanines have been discovered. These compounds have pharmacological activity especially as antihypertensive agents.

## Summary of the Invention

Fluorophenylalanines of the formula

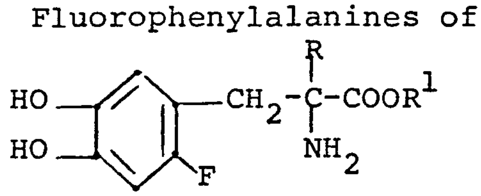

where R is H or $CH_3$ and $R^1$ is H or $C_1$-$C_{18}$ alkyl, their preparation and pharmaceutical use.

## Description of the Preferred Embodiments

An embodiment of the present invention is compounds having the formula

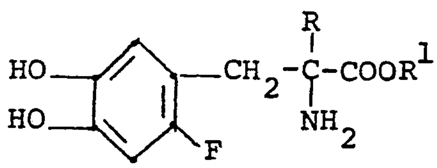

I

and pharmaceutically acceptable salts thereof wherein

R is H or $CH_3$ and

$R_1$ is H, $C_1$-$C_{18}$ alkyl or substituted $C_1$-$C_3$ alkyl.

The pharmaceutically acceptable salts of the formula I compound are the acid additional salts. Suitable salts are those with organic as well as inorganic acids. The inorganic acid salts such as the hydrohalides, and especially the hydrochlorides, are preferred.

The chiral center at the 2 carbon on the alanine moiety confers optical activity on the formula I compounds. The optical isomers may be designated as L and D, l and d, + and -, S and R or by combinations of these symbols. Where no isomer designation is indicated, the compound includes the individual isomers, mixtures thereof and racemates. The isomer designation when it appears, refers to the chiral center at the 2-carbon atom. The (S)-isomer is preferred.

The alkyl definition of $R^1$ includes linear and branched alkyls of up to 18 carbon atoms, e.g. $CH_3$, nonyl and octadecyl. The preferred alkyl groups are the lower alkyls having from 1-6 carbons such as isopropyl and *n*-hexyl, with ethyl being most preferred. The substituted $C_1$-$C_3$ alkyl $R^1$ groups include those disclosed in U.S. 4,058,530 and U.S. 3,988,341, the disclosures being

herein incorporated by reference to the extent necessary. Substituted ethyl and methyl groups are preferred, especially α-pivaloyloxyethyl, α-succinimidoethyl and β-acetamidoethyl.

A preferred group of compounds is one represented by the formula

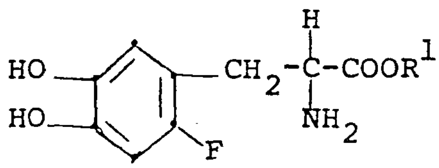

or

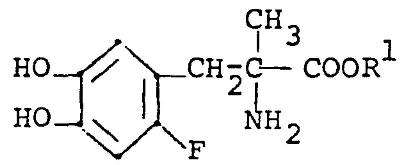

II III

More preferred are the compounds of formula II or III where $R^1$ is H, ethyl or substituted ethyl or methyl, particularly where $R^1$ is H. Especially preferred is the (S)-isomer of the formula II or III compound where $R^1$ is H; most preferred is the (S)-isomer of the formula III compound where $R^1$ is H.

The compounds of the present invention are useful as pharmaceuticals, e.g. for treatment of hypertension.

For treating hypertension in human patients, the compounds of the present invention are administered in effective amounts in appropriate dosage forms. Any suitable route of administration may be used such as oral, parenteral, intramuscular and the like. The appropriate forms are exemplified by tablets, troches, dispersions, suspension, solutions, capsules and the like for oral administration; suspensions, solutions, emulsions and the like for parenteral administration.

The daily dosage for treating hypertension in humans may range from about 10 mg to about 5,000 mg; preferably, from about 100 to about 3,500 mg; more preferably from about 200 to about 1,500 mg.

The present compounds may have decarboxylase inhibiting activity, i.e. they may interfer with the

enzyme system which effects decarboxylation of an α-amino acid, such as DOPA. The exact mechanism of action of the present compounds is not critical to the pharmaceutical utility of the compounds.

Advantages that the present compounds, and especially the more preferred ones, may offer for treating hypertension are increased potency, longer duration of action, and fewer unwanted side effects, e.g. dry mouth, drowsiness, etc.

The pharmaceutical compositions containing the compounds of the present invention comprise another embodiment of the present invention. These compositions are made using conventional preparative procedure and can contain conventional compounding ingredients, i.e. diluents, carriers, excipients, etc., as required.

The compounds of the present invention may be prepared by any convenient process.

One such process is illustrated by the following set of reaction equations:

PROCESS A

(S)

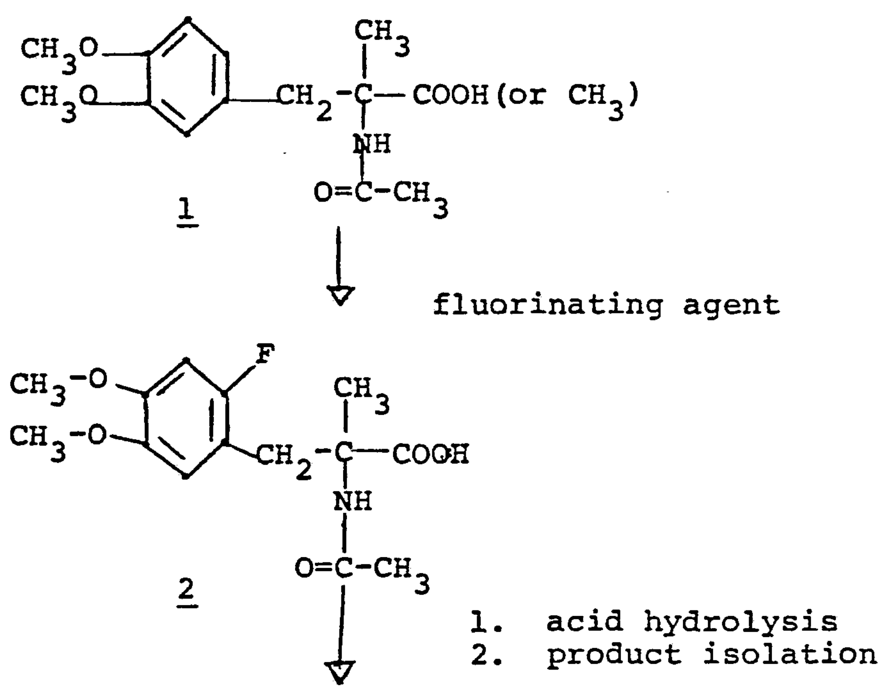


1

(S)

2

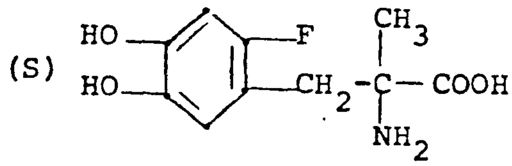

3

In process A, (S) (-)-α-methyldopa dimethylether-N-acetate (compound 1), or its methylester, is treated with a fluorinating agent such as fluorine, xenonfluoride ($XeF_2$) or fluoroxytrifluoromethane ($CF_3OF$) in liquid HF or liquid HF saturated with $BF_3$. The fluorination is ordinary-ily carried out at -80°C to 10°C. The product obtained is (S)-6-fluoro-α-methyldopa dimethylether-N-acetate (compound 2). This compound is then acid hydrolyzed and the product is isolated 3.g. by chromatography on a suitable cation exchange resin, to yield the product 3, (S)-6-fluoro-α-methyldopa.

Another process for preparing compounds of the present invention is illustrated by the following equations:

PROCESS B

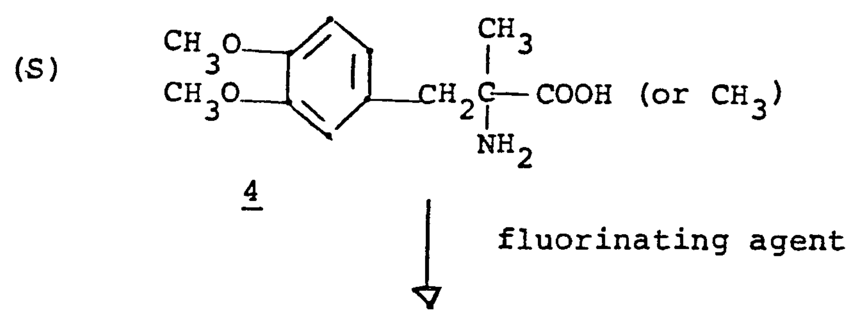


4

(S)

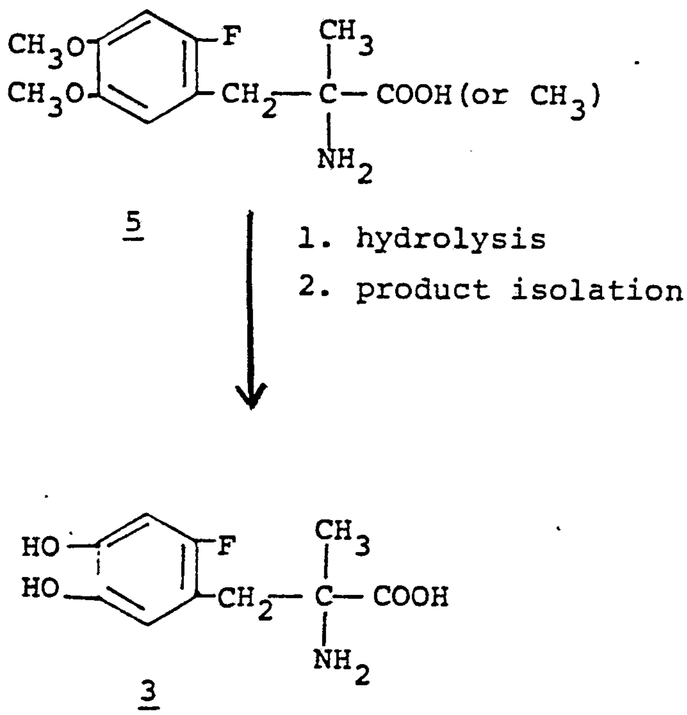


5

(S)

3

In process B, compound 4, (S)-α-methyldopa dimethylether (or its methylester) is fluorinated using the fluorinating agent as described above for process A. The product 5, (S)-6-fluoro-α-methyldopa dimethylether (or its methyl ester), is then hydrolyzed, preferrably using an acidic system, and the desired product 3, (S)-6-fluoro-α-methyldopa is isolated by conventional means, for example by chromatography on a suitable cation exchange resin.

A third process for preparing compounds of the present invention is illustrated by the following equations:

PROCESS C

(S)

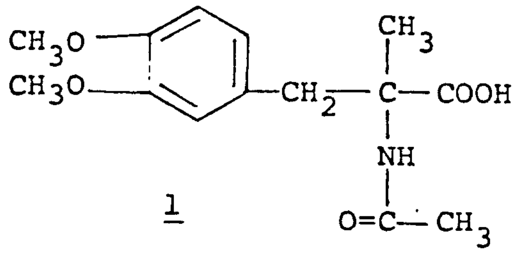

1

nitration

(S)

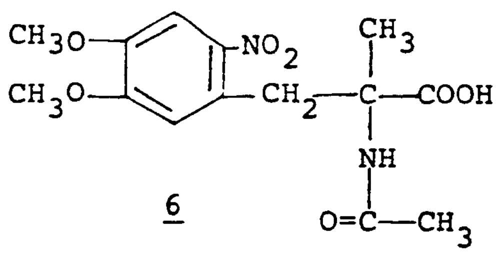

6

esterification

(S)

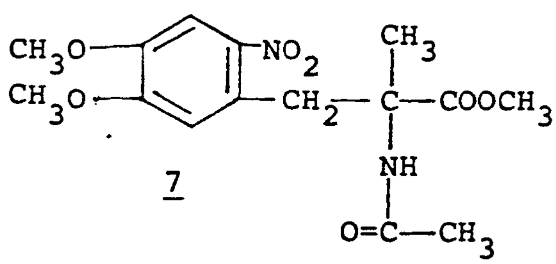

7

reduction

(S)

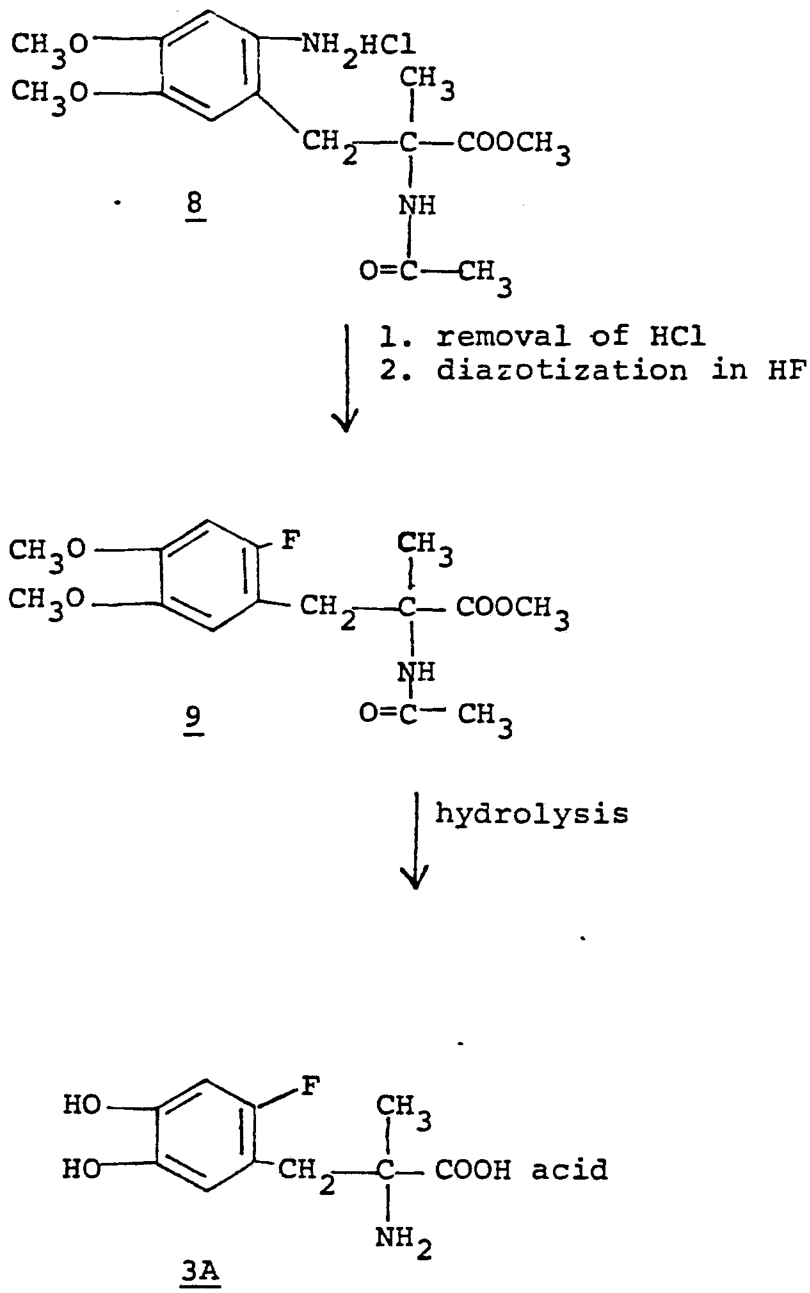


8

(S)

9

(S)

3A

Process C involves the nitration, using a conventional system e.g. $HNO_3$ in acetic acid, of compound 1 to produce 6, (S)-6-nitro-α-methyldopa dimethylether-N-acetate. Compound 6 is in turn conventionally esterified (e.g. methyl orthoformate in methanol with HCl gas catalyst).

THe ester 1 is then reduced using a suitable reducing system such as $Pd/H_2$ in acetic acid/HCl to obtain compound 8, (S)-6-amino-α-methyldopa dimethylether-N-acetate methylester·HCl. Compound 8 is treated with liquid HF to displace the HCl; the free base is then diazotized with $NaNO_2$ in HF solution using conventional conditions. The product obtained is 9, (S)-6-fluoro-α-methyldopa dimethylether-N-acetate which in turn is hydrolyzed (acid) to provide (S)-6-fluoro-α-methyldopa·acid salt (3A).

A fourth process for preparing compounds of the present invention is illustrated by the following equations:

PROCESS D

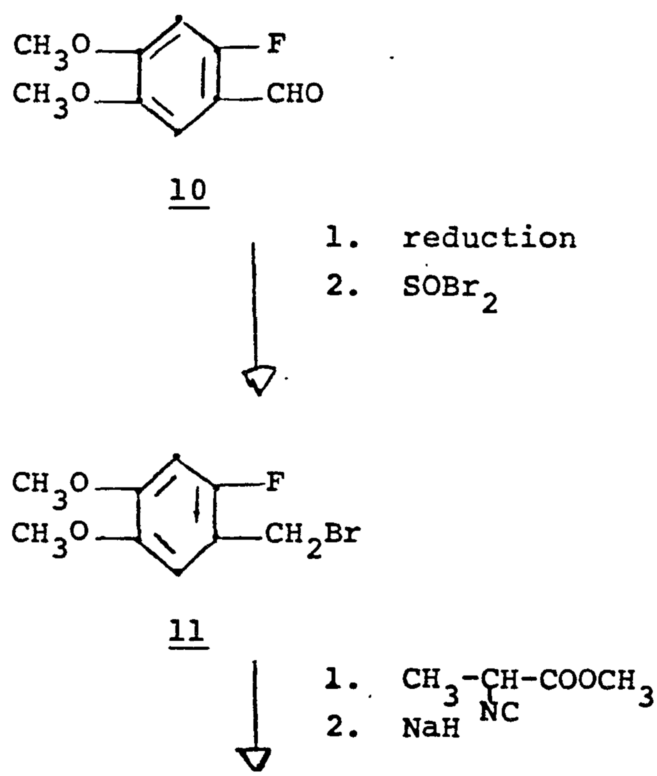

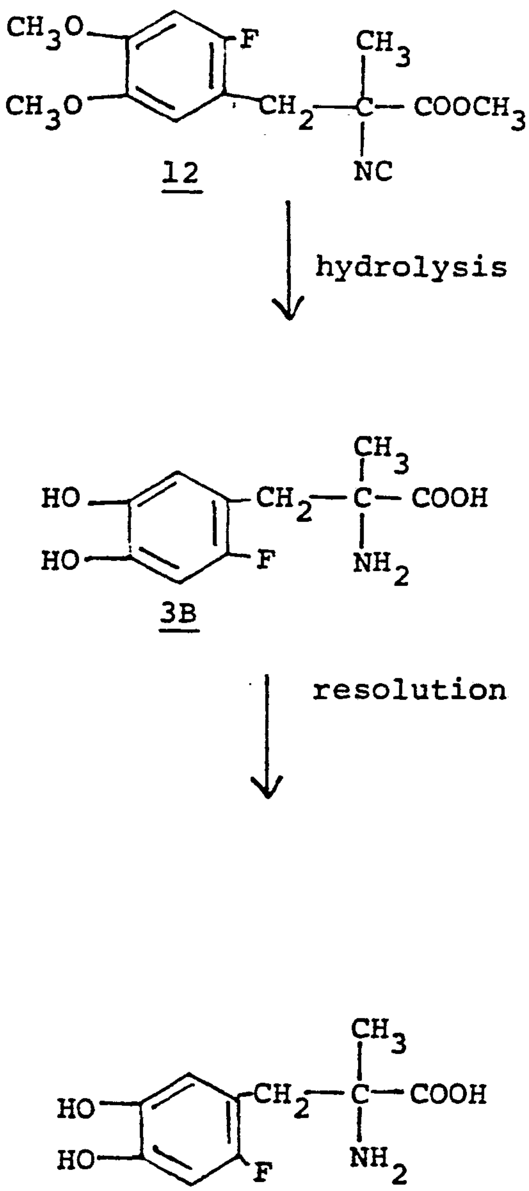


12

3B

(S)

3

In process D, 6-fluoro-3,4-dimethoxy benzaldehyde (10) (which is obtained by diazotization, in liquid HF/$NaNO_2$, of 6-amino-3,4-dimethoxybenzyl alcohol) is reduced e.g. with $NaBH_4$ and treated with $SOBr_2$ to obtain compound 11, 6-fluoro-3,4-dimethoxybenzyl bromide. Compound 11 is treated with methyl-α-isocyano propionate and a base (sodium hydride) yielding 6-fluoro-α-methyl-α-isocyanodopa

dimethyl ether methyl ester 12. This compound is in turn conventionally hydrolyzed to produce racemic 6-fluoro-α-methyldapa 3B which is resolved, using a conventional resolution procedure, to obtain the (S) enantiomer 3.

The following examples illustrate the preparation of compounds of formula I using the processes disclosed above. All temperatures are in degrees Celsius:

Example 1 (Process A)

2.82 g of S-(-)-α-methyldopa dimethylether N-acetate is dissolved in 40 ml of liquid hydrogen fluoride. The solution is cooled in a dry ice-acetone bath and while being stirred, a stream of a gaseous mixture of fluorine and helium is passed in (2% fluorine - 98% helium) in the course of 3 hours. The volume of F/He mixture employed is 11.2 l. (under STP conditions). The cooling bath is removed and the solvent is evaporated by passing in a stream of nitrogen gas. The residue is dissolved in 20 ml of 5M aq. HCl, evaporated to dryness in vacuo, the residue is redissolved in 50 ml of conc. aq. HCl and heated in a sealed tube for 3 hours in an oil bath of 130°C. The solution thus obtained is evaporated in vacuo and the residue obtained dissolved in 28 ml of water, charcoaled and subjected to elution chromatography (1.1 of Dowex-50-X-8 cation exchange resin, 200-400 mesh size). Elution with water (2 l.), IM aq. HCl (1.5 l.) and

2 Maq. HCl. The course of chromatography is followed with a recording spectrophotometer (Schoeffel SF770) set at 228 nm. The composition of the eluates is studied by $^1H$ nuclear magnetic resonance spectroscopy. A fraction of the eluate - obtained with 2M aq. HCl on evaporation <u>in vacuo</u> - consists of (S)-6-fluoro-α-methyldopa hydrochloride. For liberation of the free amino acid, this hydrochloride salt is dissolved in water and passed to an anion exchange resin column (Dowex-1, formate form); the effluent on evaporation <u>in vacuo</u> gives (S)-6-fluoro-α-methyldopa.

Example 2 (Process B)

1.2 g of (S)-α-methyldopa dimethylether is dissolved in 55 ml of liquid hydrogen fluoride. The solution is cooled in a dry ice-acetone bath and while being stirred, a stream of a gaseous mixture of fluorine and helium (2% fluorine, 98% helium, purchased from Matheson) is passed in in the course of 4-1/2 hours. The total volume of gas mixture employed is 4.1 l. STP. After removal of the cooling bath, the solvent is evaporated by passing in a stream of nitrogen gas. The residue obtained is dissolved in 25 ml of 5M aq. HCl, evaporated to dryness <u>in vacuo</u>, the residue is redissolved in 30 ml of conc. aq. HCl and heated in a sealed glass tube in an oil bath of 130°C. The hydrolysis solution thus obtained is evaporated <u>in vacuo</u> to give a residue containing (S)-6-fluoro-α-methyldopa. For purification, this is chromatographed on Dowex-X-8 cation exchange resin column in analogous manner as it is described in Example 1, to result in (S)-6-fluoro-α-methyldopa HCl salt.

The process of Example 2 can also be carried out using an appropriate amount of $CF_3OF$ in place of fluoride.

Example 3 (Process C)

3.5 g of (S)-(-)-α-methyldopa dimethylether N-acetate is suspended in 17 ml of glacial acetic acid. 6 drops of cc. $H_2SO_4$ is added, followed by a mixture of 3.5 ml of conc. nitric acid and 3.5 ml glacial acetic acid. It is heated to 50° for 15 minutes and quenched on ice. Extraction with ethyl acetate (4 x 60 ml), backwash the combined extracts with saturated NaCl solution, dry over $MgSo_4$, evaporate in vacuo and recrystallize from 66 ml of isopropanol. The crystalline product displays the correct $^1H$ NMR spectrum, with 0.25 mol of crystal isopropanol present.

For esterification, 1.30 g of the above product (about 4 mmol) is dissolved in 20 ml of methanol, 2 ml of trimethyl orthoformate is added and HCl gas is passed in with stirring and refluxing. The reaction mixture is evaporated to dryness in vacuo, and this treatment is repeated once more. The residue is dissolved in hot isopropanol. On cooling the product: (S)-6-nitro-α-methyldopa dimethylether N-acetate methyl ester crystallizes out, melting point 148-149°C.

For reduction of the nitro group, 514 mg of the above named compound is dissolved in 20 ml of glacial acetic acid, 0.3 ml of cc. HCl is added, followed by 55 mg of $PtO_2$ catalyst and hydrogenated at starting hydrogen pressure of 50 lsi gauge. The pressure drop indicates after 100 minutes the uptake of the calculated amount of hydrogen necessary for transformation of the nitro group into the amino group. The catalyst is filtered off, the filtrate evaporated in vacuo to dryness to give the hydrochloride of (S)-6-amino-α-methyldopa dimethylether N-acetate methyl ester HCl salt.

Two hundred and ninety mg of this amino derivative is charged into a small KEL-F$^R$ reactor and 5 ml of anhydrous HF liq. is condensed in the top of it. The excess and HCl are removed by passing through it a stream of dry $N_2$ gas; the residue is redissolved in 10 ml of liq. HF (cooling bath dry ice-acetone), then 50 mg of sodium nitrite is added. The cooling bath is removed and the reaction mixture let stand at room temperature overnight. The HF solvent is removed by a stream of dry $N_2$; the residue represents (S)-6-fluoro-α-methyldopa dimethylether N-acetate methyl ester. Acid hydrolysis of it (as described above) provides (S)-6-fluoro-α-methyldopa.

Example 4 (Process D)

a) 6-Fluoro-3,4-dimethoxybenzaldehyde

1.81 g of 6 amino-3,4-dimethoxybenzaldehyde is dissolved in 65 ml of liquid hydrogen fluoride. While the reactor is immersed in a dry ice-acetone bath, 0.69 g of sodium nitrite is added in 5-minute interval. The reactor is kept for 2 hours in this cooling bath, then kept in an ice bath overnight. The HF solvent is removed by blowing in a stream of nitrogen gas, the residue is quenched in ice-water and the product extracted with diethyl ether. The ether solution is washed twice with water, dried over $MgSO_4$, the solvent removed by evaporation, to give 6-fluoro-3,4-dimethoxybenzaldehyde.

b) 6-Fluoro-3,4-dimethoxybenzylalcohol

1.84 g of 6-fluoro-3,4-dimethoxybenzaldehyde is dissolved in 30 ml of ethanol, then 0.25 g of sodium borohydride is added. The reduction is over in about 30 minutes. The reaction mixture is evaporated to dryness and the residue stirred with about 45 ml of water for an hour. Extraction with diethyl ether, drying of the ether solution with $MgSO_4$, evaporation of the solvent *in vacuo* gives the title compound.

c) 6-Fluoro-3,4-dimethoxybenzylbromide

1.86 g of 6-fluoro-3,4-dimethoxybenzylalcohol is dissolved in 15 ml of toluene, then 2.08 g of thionylbromide is added. After standing at room temperature, the reaction mixture is washed with water, aq. $NaHCO_3$ solution and washed with water again, dried over $MgSO_4$ and evaporated to dryness, to give 6-fluoro-3,4-dimethoxybenzylbromide.

d) 6-Fluoro-α-methyl-α-isocyanodopadimethylether methyl ester

A suspension of sodium hydride was prepared by adding 8.64 g of 50% sodium hydride-in-oil dispersion to 200 ml of tetrahydrofuran. After 30 min. stirring a solution of a mixture of 17 g of methyl-α-isocyanopropionate and 40.6 g of 6-fluoro-3,4-dimethoxybenzyl bromide in 135 ml of tetrahydrofuran is added in a period of 50 minutes with stirring and keeping the temperature at about 25°C. The stirring is continued for 4 hours at room temperature, the mixture is brought to pH 7 by addition of dil. acetic acid (ice cooling) and evaporated to dryness *in vacuo*. The resulting residue is extracted with ethyl acetate, the extract is washed with water, dried over $MgSO_4$ and evaporated *in vacuo* to obtain the isocyano compound named above. (the above described operations - until the stage of

extraction--are conducted under a blanket of dry nitrogen gas.)

e) (±)-6-Fluoro-α-methyldopa

Twelve g of the isocyano derivative obtained above is added to 150 ml of aq. conc. HCl and heated in a sealed glass tube for 4 hours in an oil bath kept at 125°C. The solution thus obtained is evaporated to dryness, redissolved in 1M aq. HCl, charcoaled and evaporated in vacuo again. The residue is dissolved in isopropanol, then 3 ml of propylene oxide is added, to cause the precipitation of (±)-6-fluoro-α-methyldopa.

f) (S)-6-Fluoro-α-methyldopa

Optical resolution of the above racemic compound--with any of the methods employed for resolution of α-methyldopa--delivers (S)-6-fluoro-α-methyldopa.

Compounds of formula I where R is H are prepared using substantially the same procedures as illustrated in Examples 1-4 but substituting the appropriate dopa starting material. For example, in Example 1, (S)-dopa dimethylether N-acetate (or ester) of the formula

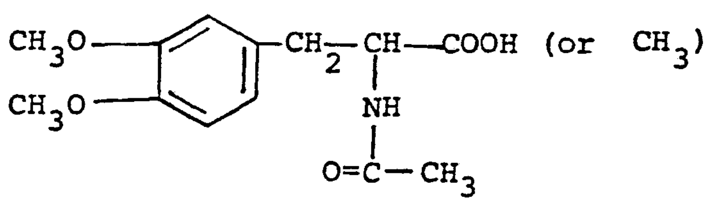

would be used in place of the corresponding α-methyl compound.

Compounds of the present invention may also be prepared by the direct fluorination of DOPA or α-methyldopa.

A fifth process for preparing compounds of formula I is illustrated by the following equations:

Process E

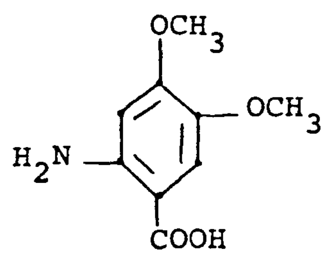

3'

$NaNO_2$ in HF/DMSO

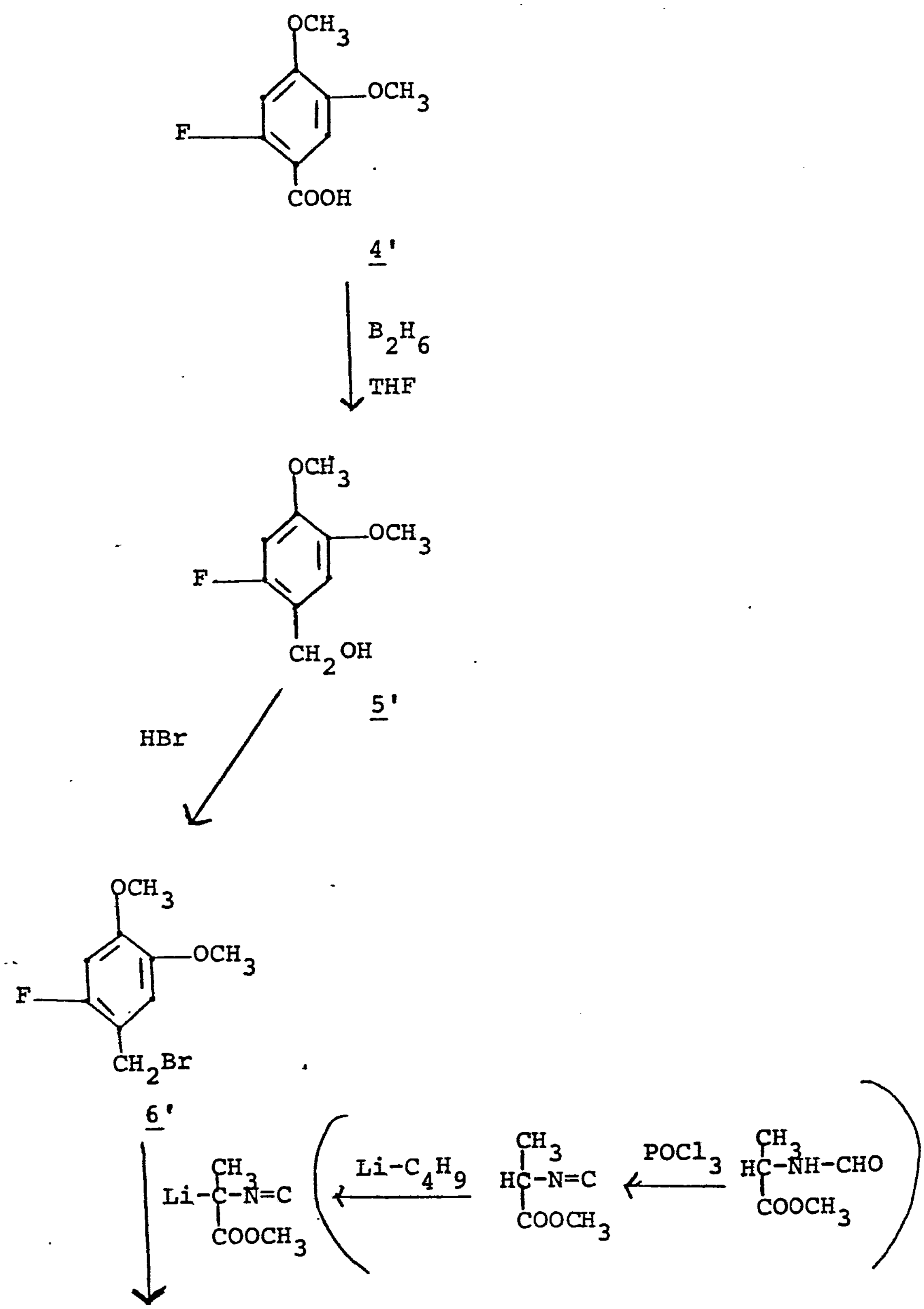


4'

5'

6'

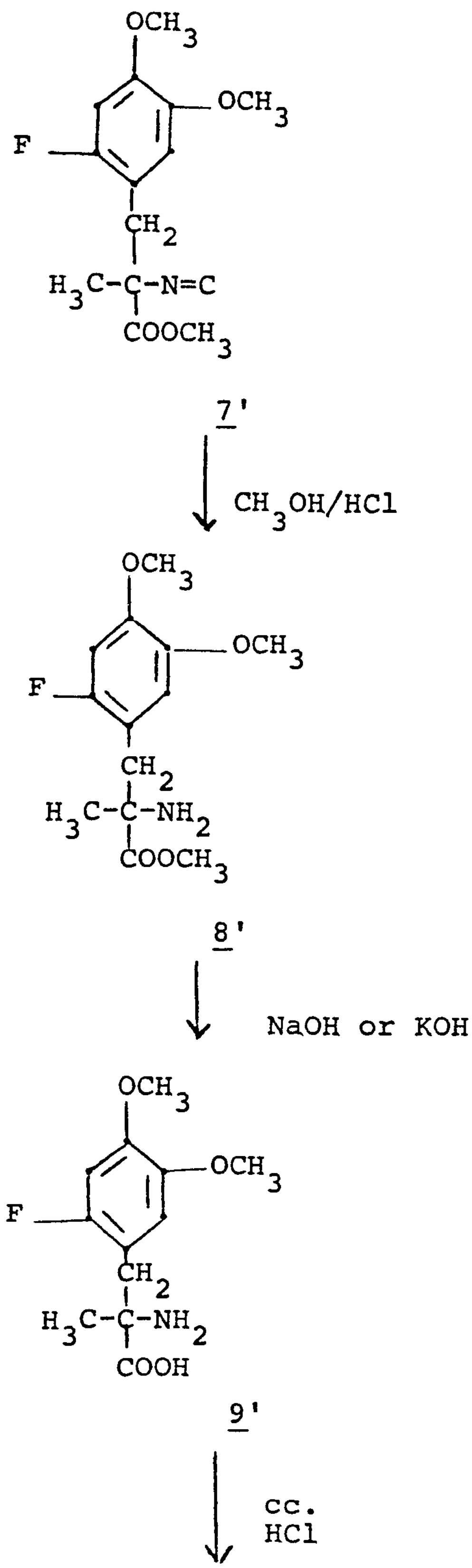
7'
$CH_3OH/HCl$
8'
NaOH or KOH
9'
cc.
HCl

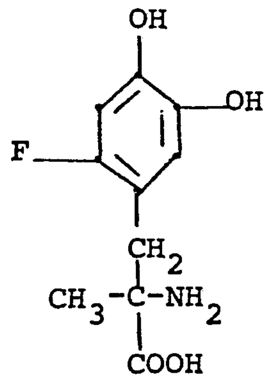

10'

A process for resolving the R/S formula 9' compound is illustrated by the following set of equations:

Resolution Process

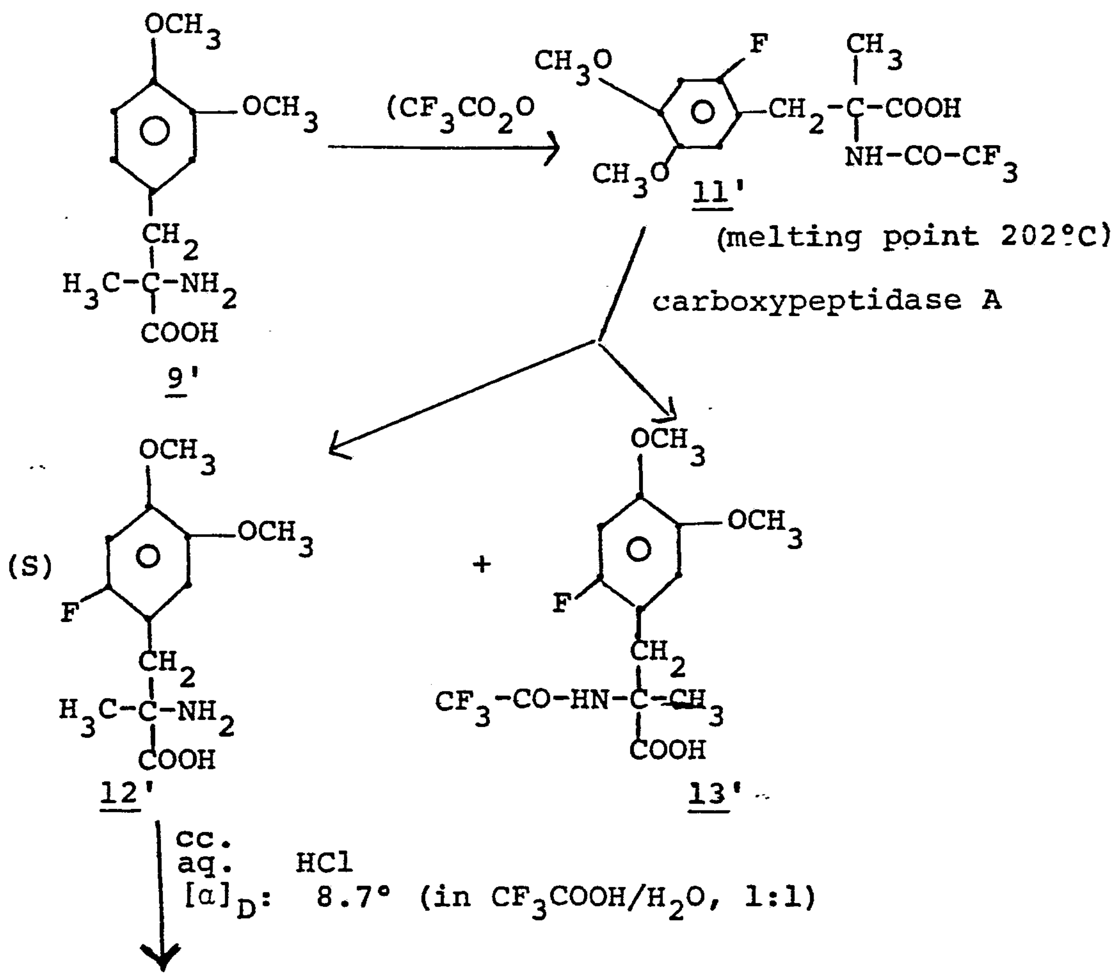


9'

11'

12'

13'

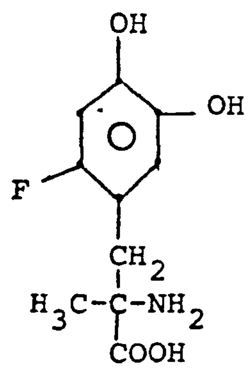

14'

(S)-6-fluoro-α-methyldopa

$[\alpha]_D$: 13.5° ($CF_3COOH/H_2O$, 1:1)

The following examples illustrate the process E and the resolution process. The underlined numbers correspond to the underlined numbers in the equations set out above. All temperatures are in degrees Celsius. Rexed means recrystallized.

Example 5 (Process E)

a) Preparation of 6-Fluoroveratric Acid 4'

Six and 9/10 g of 6-aminoveratric acid (3') was added into 30 ml of DMSO-HF (dimethyl sulfoxide - hydrogen fluoride) mixture, prepared according to procedures in United States Patents No. 3,573,214 and No. 3,471,512. The solution was cooled to -5 to 0°C and with stirring and cooling, in about 5 minutes 2.4 g of Na $NO_2$ was added. The reactor was immersed into an oil bath kept at 130-140°. After 2 hours of stirring at this temperature range, the mixture is allowed to cool to room temperature and poured onto 250 ml of ice-water mixture. After stirring overnight at room temperature the product is filtered, washed with water and dried in vacuo. For purification, rexing from isopropanol (with charcoal) is employed, followed by sublimation in vacuo (ca. 1 m/m Hg pressure). Off-white crystals of 4', m.p. 198-9°C., were obtained. (Note: The reactor employed in this experiment was made of KEL-F®. Kel-F® is a brand of polytrifluorochlorethylene made by Kellogg & Co.)

b) Preparation of 6-Fluoroveratryl alcohol 5'

Five g of 6-fluoroveratric acid was stirred in 55 ml of dry tetrahydrofuran, then cooled in an ice-water bath and under stirring ($N_2$ blanket) in about 20 minutes 37.5 ml of a tetrahydrofuran-diborane solution (1-molar) was added. After further stirring with cooling (1 hour) the excess diborane was removed by adding a 1:1 mixture of tetrahydrofuran-water. The solvents were removed by evaporation in vacuo, the residue dissolved in a mixture of diethyl ether (50 ml) and water. 6 g of $K_2CO_3$ was added and stirred for 3 hours. The layers were separated,

the aqueous phase was extracted with diethyl ether (2 x 50 ml); the combined ether solutions were back-extracted with saturated $NaHCO_3$ solution, dried over $MgSO_4$, filtered and evaporated in vacuo to give 5' as an oil. On scratching, crystals of 5', m.p. 53-55°C were obtained. A sample was rexed from hexane, m.p. 55-56°C.

c) 6-Fluoroveratryl bromide 6

Two and 234/1000 g of 6-fluoroveratryl alcohol were dissolved in 30 ml of benzene and while it was immersed into an ice-water cooling bath and stirred, hydrogen bromide gas was passed in for 15 minutes. The upper layer was poured off and the lower phase was extracted with 20 ml of benzene. The combined benzene solution was stirred with dry potassium carbonate, filtered and the filtrate evaporated in vacuo to yield crystalline 6'.

d) Methyl-3(3',4'-dimethoxy-6'-fluoro)phenyl 2-isocyano-2-methyl Propionate 7'

Two and 85/100 g of 6-fluoroveratryl bromide (6') was dissolved in 20 ml of dry tetrahydrofuran and 1.45 g of methyl α-isocyano propionate was added. This solution was added into a solution of lithium diisopropylamide (prepared previously). The addition was done at -60°C in 15 minutes. (Preparation of lithium diisopropylamide solution: into a solution of 1.93 ml of diisopropylamine in 25 ml of dry tetrahydrofuran in ca. 10 minutes 5.7 ml of 2.4 molar butyllithium solution in hexane is added, while kept at -5°C under stirring.) The lithium diisopropylamide solution was cooled, in a dry-ice/acetone bath during the addition of the earlier described solution. After 30 minutes

aging in the -78°C bath, the mixture was aged overnight at room temperature. (Note: All these operation were performed under rigorous exclusion of air, that is, under a blanket of $N_2$.) The mixture was quenched by addition of 10 ml of $H_2O$ and evaporated to dryness in vacuo. 10 ml of $H_2O$ was added again, extracted with ethyl acetate (50 ml) and the extract washed with $H_2O$ (2 x 10 ml). The combined aqueous phase was reextracted with ethyl acetate (50 ml). The ethyl acetate solution was dried ($MgSO_4$) and evaporated in vacuo, to give compound 7'.

e) 6-Fluoro-α-methyldopa dimethyl ether (9')

Compound 7', obtained above was dissolved in methanol, then 40 ml of methanolic HCl was added and stirred at 50°C for 1 hour. Evaporation in vacuo gave a gray solid (8') which dissolved in 20 ml of methanol, then a solution of 2.6 g of KOH in 30 ml of methanol was added, stirred for one hour, followed by addition of 20 ml of $H_2O$ and of 50 ml of methanol. After standing at room temperature for 60 hours, it was filtered and evaporated to dryness in vacuo. Water (50 ml) was added, then the solution extracted with diethyl ether and the aqueous layer was evaporated in vacuo to give 9'. For purification this residue was dissolved in $H_2O$, filtered and applied onto a cation exchange resin column (AG-50X8, 200/400 mesh) in the H+ form and eluted with water, then with 2N $H_2O/NH_3$. The ammonia eluate was evaporated to dryness, to give compound 9'.

f) (+)-6-Fluoro-α-methyldopa 10'

One and 35/100 g of compound 9' was charged to a Fisher-Porter tube, 30 ml conc. HCl was added, the tube sealed and heated in an oil-bath of 130°C for 1-1/2 hours.

After cooling, the tube was opened, the contents filtered and the filtrate evaporated to dryness in vacuo. The residue was dissolved in $H_2O$ (20 ml), charcoaled and evaporated again in vacuo to give the hydrochloride of 10. For isolation of the free amino acid, an aqueous solution of this was applied onto an anion exchange resin column prepared from 110 ml of AG-1 X 8 (200/400 mesh) in the formate form and eluted with water. The fractions absorbing at 254 nm were combined and evaporated to dryness to give (±)-6-fluoro-α-methyldopa (10'). It may be recrystallized from water. It was characterized by $^1H$ and $^{19}F$ NMR spectra and C/H/N/F analysis.

Example 6 (Resolution Process)

a) (+)-N-Trifluoroacetyl-6-Fluoro-α-methyldopa-dimethylether (11').

Compound 9' (386 mg) was dissolved in 3 ml of trifluoroacetic acid, then 1 ml of trifluoroacetic anhydride is added. After 1/2 hour standing at room temperature, the solution was evaporated in vacuo to dryness. 3 ml water was added, followed by evaporation to dryness. The residue was crystallized by dissolving it in 5 ml of acetone, followed by addition of 15 ml of cyclohexane. The crystals are filtered and dried, to give compound 11', mp 201-202°C.

b) (S)-6-Fluoro-α-methyldopa-dimethylether 12'

Compound 11' (2.6 g) was suspended in 50 ml water, then 50 ml of 0.1M NaOH solution was added. The solution was placed into an oil-bath, thermostated at 37°C, and under stirring the pH was adjusted to 7.2 by adding more NaOH (0.1M) solution by employing a pH-STAT (Radiometer); 12.914 ml is consumed. 1.25 ml of a carboxypeptidase

A-DFP solution (SIGMA product; 24 mg of protein/ml) was added. After stirring overnight, another similar amount of enzyme solution was added. After 3 more days stirring under similar conditions, while the pH was kept at 7.2 by the pH-STAT, ca. 3 ml more of the NaOH solution was consumed. After adding 200 mg of DARCO G-60 (brand of charcoal), it was filtered with CELITE filter aid and acidified to pH 1.5-2 and extracted with ethyl acetate (4 x 500 ml). The aqueous phase contained compound 12' whereas evaporation of the ethyl acetate extract gave compound 13'. The aqueous phase was applied onto the top of an AG50-X8 cation exchange resin column, H+, form (42 ml resin, 200/400 mesh). The column was washed with water, then the product (compound 12') was released with aqueous $NH_3$ (1 molar). Evaporation *in vacuo* gave pure 12'.

c) (S)-6-Fluoro-α-methyldopa (14')

Compound 12' (800 mg) was sealed into a Fisher-Porter tube with 10 ml of conc. aq. HCl and heated in an oil-bath for 4 hours. Evaporation *in vacuo* of the solution obtained gave the hydrochloride of compound 14'. Compound 14' was liberated from this on an AG 1-X-8 anion resin column (formate form; 45 ml resin). Elution with water and evaporation of the eluate *in vacuo* gave compound 14', for recrystallization, it was dissolved in hot $H_2O$ (3 vols/g), then isopropanol added (10 vols/g). Filtration (after standing in the ice-box overnight) gives 14'.

Claims to the invention follow.

WHAT IS CLAIMED IS

1. Compounds having the formula

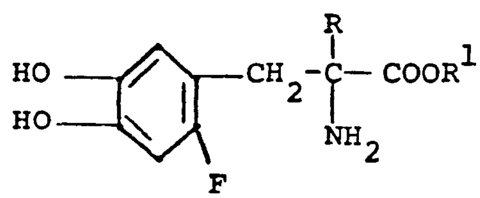

and pharmaceutically acceptable salts thereof wherein

R is H or $CH_3$ and
$R^1$ is H, $C_1$-$C_{18}$ alkyl or substituted $C_1$-$C_3$ alkyl.

2. Compound of Claim 1 wherein $R^1$ is H or lower alkyl.

3. Compound of Claim 2 wherein $R^1$ is H or ethyl.

4. Compounds of Claim 3 wherein (a) R is H, (b) R is H and $R^1$ is H, (c) R is $CH_3$, (d) R is $CH_3$ and $R^1$ is H, or (e) R is $CH_3$, $R^1$ is H and is the (S)-isomer.

5. Compounds of Claim 1 wherein (a) $R^1$ is substituted $C_1$-$C_3$ alkyl, (b) $R^1$ is α-succinimidoethyl, 2-pivaloyloxymethyl or β-acetamidoethyl, (c) $R^1$ is as in (b) and R is H, (d) $R^1$ is as in (b) and R is $CH_3$ or (e) $R^1$ is as in (b), R is $CH_3$ and is the (S) isomer.

6. A pharmaceutical composition containing a compound of Claim 1.

7. A process for preparing compounds of the formula

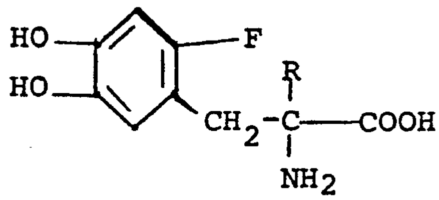

wherein R is H or $CH_3$
which comprises hydrolysis of a compound of the formula

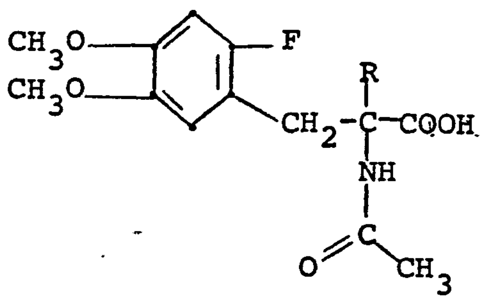
,
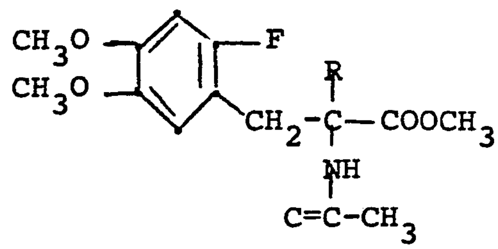

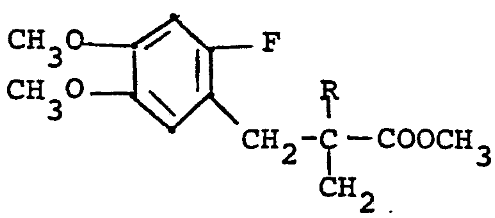
,
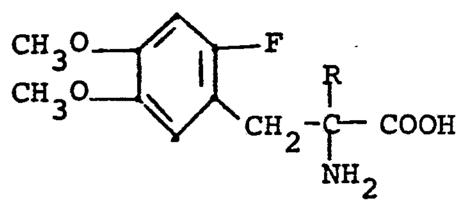

or
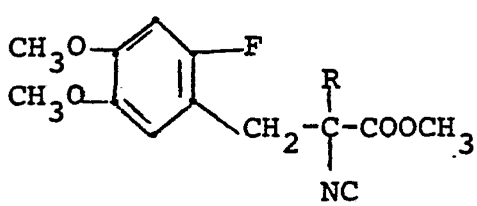

Claim for Austria

A process for preparing compounds of the formula

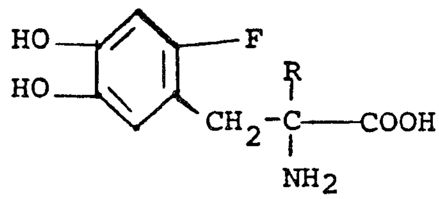

wherein R is H or $CH_3$

which comprises hydrolysis of a compound of the formula

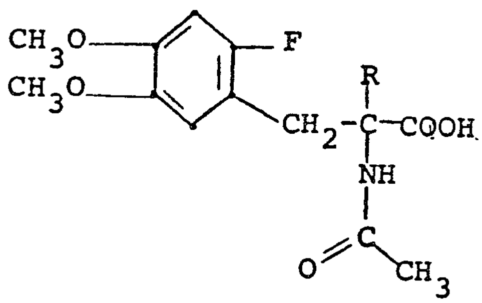

,

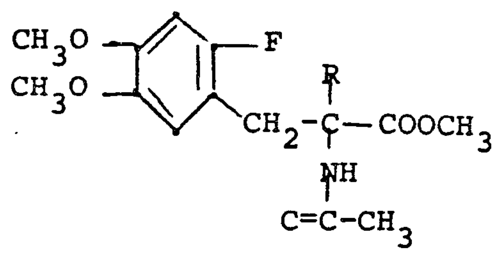

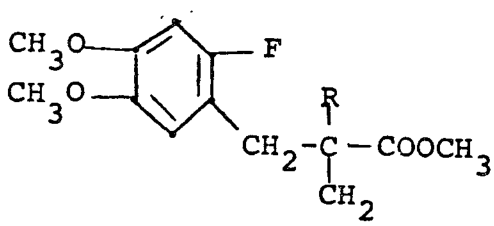

,

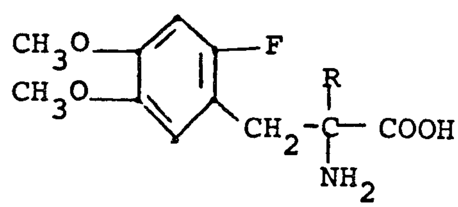

or

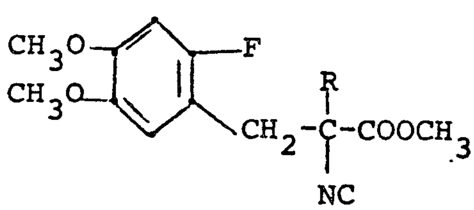

0027993

European Patent Office

# EUROPEAN SEARCH REPORT

Application number
EP 80 10 6408.0

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | US - A - 3 725 470 (BRETSCHNEIDER et al.) * example 33 * | 1-4 | C 07 C 101/77<br>C 07 C 101/72<br>A 61 K 31/195<br>A 61 K 31/215 |
| | -- | | |
| | Chemical Abstracts vol. 86, 3rd January 1977 Columbus, Ohio, USA G. FIRNAU et al. "Intracerebral dopamine metabolism studied by a novel radioisotope technique" page 180, left column, abstract no. 1928w<br>& J. Pharm. Pharmacol., vol 28, no. 7, 1976, pages 584 to 585 (Eng) | 1-4 | TECHNICAL FIELDS SEARCHED (Int. Cl.3)<br>C 07 C 101/77 |
| | -- | | |
| | Chemical Abstracts vol. 52, no. 1, 10th January 1958 Columbus, Ohio, USA C. KAISER et al. "2-Substituted derivatives of 3,4-dihydroxyphenylalanine" page 1958, column 314c<br>& J. Am. Chem. Soc., vol. 79, 1957, pages 4365 to 4370 | 1-4 | |
| | -- | | |
| D,A | US - A - 3 344 023 (D.F. REINHOLD et al.) | | |
| | ---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 28-01-1981 | PHILLIPS |

EPO Form 1503.1 06.78